(19) Europäisches Patentamt
European Patent Office
Office européen des brevets

(11) **EP 3 187 480 A1**

(12) **EUROPEAN PATENT APPLICATION**
published in accordance with Art. 153(4) EPC

(43) Date of publication:
**05.07.2017 Bulletin 2017/27**

(51) Int Cl.:
*C07C 27/16* (2006.01)     *C07C 29/48* (2006.01)
*C07C 31/125* (2006.01)     *C07C 45/40* (2006.01)
*C07C 49/04* (2006.01)     *C07C 51/34* (2006.01)
*C07C 53/126* (2006.01)     *B01F 3/04* (2006.01)
*B01J 32/00* (2006.01)     *C07B 61/00* (2006.01)

(21) Application number: **15837034.6**

(22) Date of filing: **07.08.2015**

(86) International application number:
**PCT/JP2015/072504**

(87) International publication number:
**WO 2016/031527 (03.03.2016 Gazette 2016/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **29.08.2014 JP 2014175315**

(71) Applicant: **Daicel Corporation
Osaka-shi, Osaka 530-0011 (JP)**

(72) Inventors:
• **TAKASE, Ichiro
  Himeji-shi
  Hyogo 671-1283 (JP)**
• **MURAI, Yoshiyuki
  Himeji-shi
  Hyogo 671-1283 (JP)**
• **SUZUKI, Takamasa
  Himeji-shi
  Hyogo 671-1283 (JP)**

(74) Representative: **Grünecker Patent- und
Rechtsanwälte
PartG mbB
Leopoldstraße 4
80802 München (DE)**

(54) **OXIDATION REACTOR AND PRODUCTION METHOD FOR OXIDE**

(57)     Provided is an oxidation reactor capable of oxidizing hydrocarbons with both good reaction efficiency and good energy efficiency. This oxidation reactor includes a liquid inlet channel, a gas inlet channel, a gas-liquid mixing unit, and a flow reactor. Through the liquid inlet channel, a liquid containing a reaction substrate hydrocarbon is introduced. Through the gas inlet channel, a gas containing oxygen and ozone is introduced. The gas-liquid mixing unit mixes the liquid introduced from the liquid inlet channel with the gas introduced from the gas inlet channel. In the flow reactor, an oxidation catalyst is immobilized or packed. In the oxidation reactor, the flow reactor includes a monolith support and the oxidation catalyst immobilized to or packed in the monolith support. In addition or alternatively, the gas-liquid mixing unit includes a microbubble generator.

[Fig. 1]

# Description

## Technical Field

The present invention relates to oxidation reactors, and methods for producing oxides (oxidation products) using the oxidation reactors. More specifically, the present invention relates to an oxidation reactor which is for use in oxidation of an organic substrate hydrocarbon in the presence of oxygen and ozone to give an oxide or oxides; and to a method for producing an oxide using the oxidation reactor. This application claims priority to Japanese Patent Application No. 2014-175315, filed August 29, 2014 to Japan, the entire contents of which are incorporated herein by reference.

## Background Art

Oxidation reactions are one of most basic reactions in the organic chemical industry, for which a variety of oxidation methods have been developed. Patent Literature (PTL) 1 describes an oxidation method by which a compound capable of forming a radical is oxidized typically with molecular oxygen by the catalysis of a lipid-soluble imide compound. With this method, a wide variety of organic substrates can be oxidized to give corresponding oxides. Disadvantageously, however, the method offers low yields when employing hydrocarbons such as straight chain alkanes as the substrate.

An oxidation reaction of a hydrocarbon with oxygen is generally performed by introducing the starting material hydrocarbon into a stirred-tank reactor, and blowing oxygen or air through nozzles into the reactor. This method, however, offers poor heat transfer and low gas-liquid mixing efficiency and is not always satisfactory in reaction efficiency and energy efficiency.

## Citation List

## Patent Literature

PTL 1: Japanese Unexamined Patent Application Publication (JP-A) No. 2002-331242

## Summary of Invention

## Technical Problem

Accordingly, the present invention has an object to provide an oxidation reactor that is capable of oxidizing hydrocarbons with both good reaction efficiency and good energy efficiency.

The present invention has another object to provide a method for efficiently producing a corresponding oxide or oxides from a hydrocarbon.

## Solution to Problem

After intensive investigations to achieve the objects, the inventors of the present invention found that a specific oxidation reactor enables efficient oxidation of a hydrocarbon. This oxidation reactor includes a liquid inlet channel to introduce the hydrocarbon, a gas inlet channel to introduce oxygen and ozone, a gas-liquid mixing unit to mix the liquid with the gases, and a flow reactor in which an oxidation catalyst is immobilized or packed. In the oxidation reactor, the flow reactor is a monolithic catalyst reactor in which the oxidation catalyst is immobilized or packed; and/or the gas-liquid mixing unit includes a microbubble generator. The present invention has been made based on these findings.

Specifically, the present invention relates to the followings.

(1) The present invention relates to an oxidation reactor that includes a liquid inlet channel, a gas inlet channel, a gas-liquid mixing unit, and a flow reactor. Through the liquid inlet channel, a liquid containing a reaction substrate hydrocarbon is introduced. Through the gas inlet channel, a gas containing oxygen and ozone is introduced. The gas-liquid mixing unit mixes the liquid introduced from the liquid inlet channel with the gas introduced from the gas inlet channel. In the flow reactor, an oxidation catalyst is immobilized or packed. In the oxidation reactor, the flow reactor includes a monolith support, and the oxidation catalyst immobilized to or packed in the monolith support. In addition or alternatively, the gas-liquid mixing unit includes a microbubble generator.

(2) The oxidation reactor according to (1) may further include a gas-liquid separator downstream from the flow reactor.

(3) The oxidation reactor according to (2) may further include a circulation channel through which at least part of a liquid separated in the gas-liquid separator is recycled to the gas-liquid mixing unit or to a portion upstream from the gas-liquid mixing unit.

(4) The oxidation reactor according to one of (2) and (3) may further include a circulation channel through which at least part of a gas separated in the gas-liquid separator is recycled to the gas-liquid mixing unit or to a portion upstream from the gas-liquid mixing unit.

(5) In the oxidation reactor according to any one of (1) to (4), the oxidation catalyst may be a catalyst including a transition metal in the form of an elementary substance, a compound, or an ion, and an inorganic support onto which the transition metal is supported or immobilized.

(6) In the oxidation reactor according to any one of (1) to (4), the oxidation catalyst may be selected from a catalyst including a transition metal compound and a support having a Hammett acidity function ($H_0$) of -9 or less and supporting the transition metal compound; and a catalyst including a transition metal ion and a support having a Hammett acidity function ($H_0$) of -9 or less and being ion-exchanged with the transition metal ion.

(7) In the oxidation reactor according to (6), the support may include a strongly acidic or super acidic ion exchange resin.

(8) In the oxidation reactor according to any one of (1) to (7), a gas-liquid mixture channel portion (space portion) in the monolith support may have an inside diameter (bore diameter) of 0.5 to 25 mm and a length of 0.05 to 20 m.

(9) In the oxidation reactor according to any one of (1) to (4) and (6) to (8), the flow reactor bears, on its inner wall, a coating of a strongly acidic or super acidic ion exchange resin being ion-exchanged with a transition metal ion.

(10) In the oxidation reactor according to any one of (6) to (9), the transition metal compound may include a compound containing at least one transition metal selected from the group consisting of Co (cobalt), Mn (manganese), Fe (iron), Zr (zirconium), Ce (cerium), V (vanadium), and Mo (molybdenum).

(11) In the oxidation reactor according to any one of (6) to (10), the transition metal compound may be at least one compound selected from the group consisting of cobalt compounds and manganese compounds.

(12) In the oxidation reactor according to any one of (6) to (11), the transition metal ion may be at least one ion selected from the group consisting of cobalt ions, manganese ions, iron ions, zirconium ions, and cerium ions.

(13) In the oxidation reactor according to any one of (6) to (12), the transition metal ion is a cobalt ion, or a manganese ion, or both (a cobalt ion and a manganese ion in combination).

(14) In the oxidation reactor according to any one of (1) to (13), the reaction substrate may be a hydrocarbon.

(15) In the oxidation reactor according to (14), the hydrocarbon may be at least one hydrocarbon selected from the group consisting of aliphatic hydrocarbons, alicyclic hydrocarbons, and aromatic hydrocarbons.

(16) The oxidation reactor according to any one of (1) to (15) may further include an imide compound inlet channel upstream from the flow reactor, where an imide compound having a cyclic imide skeleton is introduced through the imide compound inlet channel.

(17) The present invention also relates to a method for producing an oxide. The method includes oxidizing a hydrocarbon in the coexistence of oxygen and ozone using the oxidation reactor according to any one of (1) to (16), to yield a corresponding oxide or oxides.

Advantageous Effects of Invention

According to the present invention, a monolithic catalyst reactor is used as the flow reactor, where the monolithic catalyst reactor includes a monolith support, and an oxidation catalyst immobilized to or packed in the monolith support. In addition or alternatively, the gas-liquid mixing unit includes a microbubble generator. Further, both oxygen and ozone are used as oxidizers. This configuration allows reaction components to move quickly and allows oxidation to proceed rapidly at a high rate. Accordingly, with the present invention, a wide variety of hydrocarbons, in particular even straight chain alkanes, can be efficiently oxidized. The reaction heat can be efficiently removed, and high energy efficiency is obtained, because of smooth heat exchange. In addition and advantageously, the oxidation apparatus can be downsized.

Brief Description of Drawings

Fig. 1 is a schematic flow diagram illustrating an oxidation reactor according to an embodiment of the present invention; and
Fig. 2 is a schematic view of a microbubble generator according to an embodiment used as a gas-liquid mixing unit.

Description of Embodiments

Oxidation Reactor

The oxidation reactor according to the present invention includes a liquid inlet channel, a gas inlet channel, a gas-liquid mixing unit, and a flow reactor. Through the liquid inlet channel, a liquid containing a reaction substrate hydrocarbon is introduced. Through the gas inlet channel, a gas containing oxygen and ozone is introduced. The gas-liquid mixing unit mixes the liquid introduced from the liquid inlet channel with the gas introduced from the gas inlet channel. In the flow reactor, an oxidation catalyst is immobilized or packed. It is important in the oxidation reactor according to the present invention that the flow reactor is a monolithic catalyst reactor including a monolith support (monolithic support) and an oxidation catalyst immobilized to or packed in the monolith support; and/or that the gas-liquid mixing unit includes a microbubble generator. The oxidation reactor has only to meet at least one of the conditions that the flow reactor is a monolithic catalyst reactor; and that the gas-liquid mixing unit includes a microbubble generator.

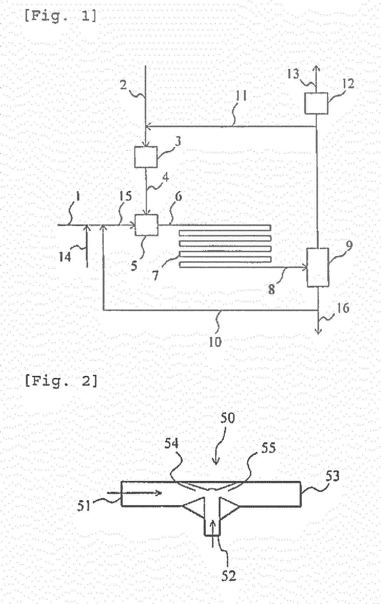

Fig. 1 is a schematic flow diagram illustrating an oxidation reactor according to an embodiment of the present invention. Fig. 2 is a schematic view of a microbubble generator according to an embodiment, which is used as a gas-liquid mixing unit 5 illustrated in Fig. 1.

In this embodiment, a reaction substrate hydrocarbon is fed through a hydrocarbon supply channel 1 to the gas-liquid mixing unit (gas-liquid mixer) 5. The hydrocarbon may be fed in the form of a solution in a solvent, as needed. On the other hand, oxygen or an oxygen-containing gas (such as air) is fed through an oxygen inlet channel 2 to an ozone generator 3 and partially converted into ozone to form a mixed gas containing oxygen and ozone, and the mixed gas is fed through a gas inlet channel 4 to the gas-liquid mixing unit 5. The mixed gas containing oxygen and ozone may be mixed with an inert gas such as nitrogen gas before being fed to the gas-liquid mixing unit 5.

The gas-liquid mixing unit (gas-liquid mixer) 5 is not limited, as long as having such a structure as to mix the gas with the liquid to form a gas-liquid mixture, but is preferably a microbubble generator. This is preferred from the viewpoints typically of mixing efficiency between the gas and the liquid and dispersibility of bubbles in the liquid. As used herein, the term "microbubble" refers to a bubble having a diameter upon generation of 50 μm or less (and generally 10 to 40 μm). Such microbubbles containing both oxygen and ozone are readily dissolved in the liquid, and this allows the oxidation reaction to proceed efficiently.

Such microbubble generators are broadly classified as those of pressurized dissolution system and those of two-phase swirling flow system. The microbubble generator according to the present invention can be selected from microbubble generators of any system. The microbubble generator can also be selected from commercially available microbubble generators (microbubble generating devices). Fig. 2 is schematic view of a microbubble generator according to an embodiment, when viewed laterally. The microbubble generator 50 includes or has an upstream side (liquid inlet side) 51, an inlet port (gas inlet side) 52, a downstream side (microbubble-containing liquid outlet side) 53, a nozzle 54, and a diffuser 55. The arrows in the figure indicate introduction directions of the liquid and the gas. The microbubble generator includes a cylindrical channel extending from the upstream side 51 to the downstream side 53, which channel is narrowed in inside diameter at a midpoint thereof. To the narrowed portion (the tip of the nozzle 54), a gas channel from the inlet port 52 is perpendicularly coupled. The liquid introduced from the upstream side 51, with the gas (mixed gas containing oxygen and ozone) introduced from the inlet port 52, flows from the nozzle 54 through the most-narrowed portion and gushes forth toward the diffuser 55 to form a microbubble-containing liquid. The formed microbubble-containing liquid is discharged from the downstream side 53. The reactor may further include a reservoir in a portion toward which the downstream side 53 is discharged, where water at rest is stored in the reservoir. In the microbubble generator, the liquid inlet channel of the upstream side 51, and the gas-liquid mixture outlet channel of the downstream side 53 may have inside diameters each independently of typically 10 to 1000 mm, and preferably 15 to 350 mm, where the inside diameters can be set as appropriate in consideration typically of microbubble generation efficiency. The gas inlet channel of the inlet port 52 may have an inside diameter of typically 2 to 300 mm, and preferably 4 to 150 mm, where the inside diameter can be set as appropriate in consideration typically of microbubble generation efficiency.

The gas-liquid mixing unit 5 is coupled (typically at the gas-liquid mixture outlet channel in the downstream side 53 of the microbubble generator 50) to a gas-liquid mixture channel 6, which leads to a flow reactor 7. The gas-liquid mixing unit 5 may be coupled directly to the flow reactor 7.

A material to constitute the gas-liquid mixing unit 5 is not limited, as long as being inert to and insoluble in the gas and the liquid to be used in the reaction. Non-limiting examples of the material usable herein include resins exemplified by fluorocarbon resins such as Teflon®, Daiflon, and poly(vinylidene fluoride)s, polyester resins, polyolefin resins, polycarbonate resins, polystyrene resins, polyamide resins, and polyimide resins; glass; and metals exemplified by titanium, and alloys such as stainless steels. The gas-liquid mixing unit 5, when made typically of a metal, may bear a resin coating and/or glass lining in a portion to be in contact with the gas-liquid mixture.

The mixed gas containing oxygen and ozone, and the liquid containing the substrate hydrocarbon, both fed to the gas-liquid mixer 5, are converted into a gas-liquid mixture in the gas-liquid mixer and discharged out, and the discharged mixture flows through the gas-liquid mixture channel 6 into the flow reactor 7. The substrate hydrocarbon is oxidized to form a corresponding oxide or oxides (oxidation product or products) in the flow reactor 7. Non-limiting examples of the oxides include alcohols, ketones, aldehydes, carboxylic acids, and hydroperoxides.

In the flow reactor 7, an after-mentioned oxidation catalyst is immobilized or packed. A material to constitute the flow reactor is not limited, as long as inert to and insoluble in the gas and the liquid to be used in the reaction. Non-limiting examples of the material include resins exemplified by fluorocarbon resins such as Teflon® and Daiflon, polyester resins, polyolefin resins, polycarbonate resins, polystyrene resins, polyamide resins, and polyimide resins; inorganic oxides such as silica; glass; and metals exemplified by titanium, and alloys such as stainless steels. The flow reactor 7, when made typically of a metal, may bear a resin coating and/or a glass lining in a portion to be in contact with the gas-liquid mixture.

The flow reactor 7 may have an inside diameter of typically 0.3 to 3 mm, preferably 0.4 to 1.8 mm, and more preferably 0.7 to 1.6 mm when the flow reactor is not an after-mentioned monolithic catalyst reactor. When the flow reactor bears, on its inner wall, a coating typically of a catalyst layer, the term "inside diameter" refers to the diameter of space excluding the coating. The flow reactor 7 may have a length of generally about 0.1 to about 20 m, and preferably about 0.3 to about 10 m, where the length can be selected as appropriate according typically to the reaction rate.

The temperature control of the flow reactor can be performed typically by configuring the flow reactor to have a multiple-tube structure and allowing a heat medium or coolant to pass through an inner tube or outer tube; or by immersing the flow reactor in a heat medium bath or coolant bath.

A reaction mixture discharged from the flow reactor 7 is introduced through a reaction mixture channel 8 into a gas-liquid separator 9 and is separated into a gas and a liquid. The flow reactor 7 may be coupled directly to the gas-liquid separator 9. The separated gas is introduced into an ozone decomposer 12 and then discharged through an exhaust gas line 13 to the outside. At least part of the separated gas is, as needed, returned through a gas circulation channel 11 typically to a portion upstream from the ozone generator 3 to thereby be recycled to the reaction system. Independently, at least part of the liquid separated in the gas-liquid separator 9 can be recycled through a liquid circulation channel 10 and a liquid inlet channel 15 to the gas-liquid mixing unit 5, typically using a pump. The liquid separated in the gas-liquid separator 9 can also be recovered through a liquid recovery channel 16. The oxidation reactor may further include, as needed, an imide compound inlet channel 14 through which an imide compound having a cyclic imide skeleton is introduced. The imide compound inlet channel 14 may be disposed upstream from the flow reactor 7, preferably at the gas-liquid mixing unit 5 or at a portion upstream from the gas-liquid mixing unit 5.

The flow reactor for use in the present invention may be a monolithic catalyst reactor which includes a monolith support, and an oxidation catalyst immobilized to or packed in the monolith support. The term "monolith" refers to a structure that includes a skeleton (material portion) and space (channel portion) continuously integrated with each other and is also called a "co-continuous structure". The space is not a closed hole, but a continuous through hole (open hole). In addition, the monolithic catalyst reactor offers significantly better gas-liquid contact efficiency, because the reactor of this type allows a slug flow and/or a Taylor flow each including gas portions and liquid portions alternately to be readily formed. Accordingly, the gas-liquid mixing unit 5 does not always have to be a microbubble generator. As described above, the use of the monolithic catalyst reactor contributes to significantly better gas-liquid contact efficiency and contact efficiency between the gas-liquid mixture and the catalyst and to significantly better oxidation reaction efficiency. In addition, the use offers easy removal of reaction heat and better energy efficiency.

A material to constitute the monolith support may be selected from any materials such as inorganic oxides (such as silica), glass, resins, and metals. The metals may have undergone resin coating and/or glass lining.

The monolith support has an inside diameter (bore diameter) in the gas-liquid mixture channel portion (space portion) of typically 0.5 to 25 mm, preferably 0.8 to 10 mm, and more preferably 0.8 to 5 mm. The monolith support has a length of generally about 0.05 to about 20 m, and preferably about 0.05 to about 1 m, where the length can be selected as appropriate according typically to the reaction rate.

In the monolith support, the gas-liquid mixture channel portion (space portion) may have any cross-sectional shape not limited, such as a triangular, rectangular, pentagonal, hexagonal, circular, or indefinite shape. The monolith support may be selected typically from commercially available polymer monolithic columns.

Oxygen and Ozone

The present invention uses the oxidizer oxygen in coexistence with ozone. A mixed gas containing oxygen gas and ozone gas is used in the reaction. The combination use of the oxidizer oxygen with ozone can promote withdrawal of hydrogen from the substrate hydrocarbon to activate the radical reaction. This can promote the oxidation reaction even under mild conditions. From the viewpoints of reactivity and economic efficiency, the mixed gas containing oxygen gas and ozone gas may contain the ozone gas in an amount of typically about 0.1 to 10 volume percent relative to the oxygen gas. The oxygen and ozone may be diluted to an appropriate concentration with an inert gas such as nitrogen gas, before use. The mixed gas containing oxygen gas and ozone gas contains the oxygen gas in a concentration of typically 5 volume percent or more, preferably 10 volume percent or more, and more preferably 15 volume percent or more. The upper limit of the concentration is typically 99.9 volume percent, preferably 80 volume percent, more preferably 50 volume percent, and particularly preferably 25 volume percent.

Reaction Substrate

The present invention uses a hydrocarbon as the reaction substrate. Non-limiting examples of the hydrocarbon include aliphatic hydrocarbons, alicyclic hydrocarbons, and aromatic hydrocarbons.

Non-limiting examples of the aliphatic hydrocarbons include straight chain alkanes such as ethane, propane, butane, pentane, hexane, heptane, octane, nonane, decane, undecane, dodecane, tridecane, tetradecane, pentadecane, hexadecane, heptadecane, octadecane, nonadecane, icosane, henicosane, docosane, triacontane, and tetracontane, of which $C_3$-$C_{30}$, preferably $C_4$-$C_{20}$, straight chain alkanes are typified; branched chain alkanes such as 2-methylpropane, 2-methylbutane, 2,2-dimethylpropane, 2-methylpentane, 3-methylpentane, 2,3-dimethylbutane, 2-methylhexane, 3-methylhexane, 3,4-dimethylhexane, and 3-methyloctane, of which $C_3$-$C_{30}$, preferably $C_4$-$C_{20}$, branched chain alkanes are typified; and straight or branched chain alkenes or alkadienes such as propylene, isobutylene, 1-pentene, 1-hexene, 2-hexene, 1-heptene, 1-octene, 1-decene, 1-dodecene, 1-tetradecene, and 1,4-hexadiene, of which $C_3$-$C_{30}$, preferably $C_4$-$C_{20}$, straight or branched chain alkenes or alkadienes are typified.

Non-limiting examples of the alicyclic hydrocarbons include cycloalkanes such as cyclopropane, cyclobutane, cyclopentane, cyclohexane, cycloheptane, cyclooctane, cyclononane, cyclodecane, cyclododecane, cyclotetradecane, cyclohexadecane, and cyclotriacontane, of which cycloalkanes containing 3 to 30 members (preferably 5 to 30 members, and particularly preferably 5 to 20 members) are typified; cycloalkenes such as cyclopropene, cyclobutene, cyclopentene, cyclooctene, cyclohexene, cycloheptene, and cyclododecene, of which cycloalkenes containing 3 to 30 members (preferably 3 to 20 members, and particularly preferably 3 to 12 members) are typified; cycloalkadienes such as cyclopentadiene, 1,3-cyclohexadiene, and 1,5-cyclooctadiene; cycloalkatrienes such as cyclooctatriene; and bicyclic, tricyclic, or tetracyclic bridged hydrocarbons such as decalin (decahydronaphthalene), bicyclo[2.2.0]hexane, bicyclo[2.2.2]octane, bicyclo[3.2.1]octane, bicyclo[4.3.2]undecane, bicyclo[3.3.3]undecane, norbornane, norbornene, tricyclo[5.2.1.0$^{2,6}$]decane, tricyclo[6.2.1.0$^{2,7}$]undecane, adamantane, perhydroanthracene, perhydroacenaphthene, perhydrophenanthrene, perhydrophenalene, and perhydroindene.

Non-limiting examples of the aromatic hydrocarbons include $C_6$-$C_{20}$ aromatic hydrocarbons such as benzene, naphthalene, and anthracene.

The hydrocarbon may have, as substituents, one or more groups selected from the group consisting of aliphatic hydrocarbon groups, alicyclic hydrocarbon groups, and aromatic hydrocarbon groups.

Non-limiting examples of the aliphatic hydrocarbon groups include monovalent or higher-valent aliphatic hydrocarbon groups which are groups resulting from removing one or more hydrogen atoms each from the structural formulae of the aliphatic hydrocarbons. Non-limiting examples of the monovalent aliphatic hydrocarbon groups include $C_1$-$C_{10}$ straight or branched chain alkyls such as methyl, ethyl, propyl, isopropyl, butyl, isobutyl, s-butyl, t-butyl, pentyl, hexyl, octyl, and decyl; $C_2$-$C_{10}$ alkenyls such as vinyl, isopropenyl, and 1-butenyl; and $C_2$-$C_{10}$ alkynyls such as ethynyl and propynyl. Non-limiting examples of divalent aliphatic hydrocarbon groups include $C_1$-$C_{10}$ straight or branched chain alkylene groups such as methylene, ethylene, propylene, trimethylene, isopropylidene, and tetramethylene groups. Non-limiting examples of trivalent aliphatic hydrocarbon groups include $C_1$-$C_{10}$ alkanetriyls such as 1,2,3-propanetriyl.

Non-limiting examples of the alicyclic hydrocarbon groups include monovalent or higher-valent alicyclic hydrocarbon groups which are groups resulting from removing one or more hydrogen atoms each from the structural formulae of the alicyclic hydrocarbons. Non-limiting examples of the alicyclic hydrocarbon groups include cycloalkyls such as cyclopentyl and cyclohexyl; cycloalkenyls such as cyclopentenyl and cyclohexenyl; and bridged hydrocarbon groups such as adamant-1-yl, norborn-2-yl, and norbornane-7,7-diyl.

Examples of the aromatic hydrocarbon groups include monovalent or higher-valent aromatic hydrocarbon groups which are groups resulting from removing one or more hydrogen atoms each from the structural formulae of the aromatic hydrocarbons. Non-limiting examples of the aromatic hydrocarbon groups include phenyl, 1-naphthyl, 1,2-phenylene, 1,3-phenylene, and 1,4-phenylene groups.

The "hydrocarbon" includes compounds containing an alicyclic hydrocarbon and an aromatic hydrocarbon fused to each other in such a manner as to have two carbon atoms in common. Non-limiting examples of the compounds of this category include indane, tetrahydronaphthalene, fluorene, and acenaphthene.

The hydrocarbon may have one or more substituents other than hydrocarbon groups, within ranges not adversely affecting the reaction, or not.

The hydrocarbon for use as the reaction substrate in the present invention contains carbon atoms in a number of preferably about 2 to about 30, more preferably 3 to 25, and furthermore preferably 4 to 20, where the number of carbon atoms is not limited.

Preferred, but non-limiting examples of the hydrocarbon for use in the present invention include straight chain alkanes such as propane, butane, pentane, hexane, heptane, octane, nonane, decane, undecane, dodecane, tridecane, tetradecane, pentadecane, hexadecane, heptadecane, octadecane, nonadecane, and icosane, of which $C_3$-$C_{30}$, preferably $C_4$-$C_{20}$, straight chain alkanes are typified; branched chain alkanes such as 2-methylpropane, 2-methylbutane, 2,2-dimethylpropane, 2-methylpentane, 3-methylpentane, 2,3-dimethylbutane, 2-methylhexane, 3-methylhexane, 3,4-dimethylhexane, and 3-methyloctane, of which $C_3$-$C_{30}$, preferably $C_4$-$C_{20}$, branched chain alkanes are typified; cycloalkanes such as cyclopentane and cyclohexane, of which cycloalkanes containing 3 to 30 members (preferably 5 to 30 members, and particularly preferably 5 to 20 members) are typified; aromatic hydrocarbons each containing an aromatic ring and one or more alkyls (such as $C_1$-$C_6$ alkyls) bonded to the aromatic ring, such as toluene, o-xylene, m-xylene, p-xylene, o-t-butyltoluene, m-t-butyltoluene, p-t-butyltoluene, 1-ethyl-4-methylbenzene, 1-ethyl-3-methylbenzene, 1-isopropyl-4-methylbenzene, 1-t-butyl-4-methylbenzene, mesitylene, pseudocumene, durene, methylnaphthalene, dimethylnaphthalene, methylanthracene, 4,4'-dimethylbiphenyl, ethylbenzene, propylbenzene, butylbenzene, and 1,4-diethylbenzene; and compounds containing an alicyclic hydrocarbon and an aromatic hydrocarbon fused to each other in such a manner as to have two carbon atoms in common, such as tetrahydronaphthalene and fluorene.

In particular, the present invention is especially useful in oxidation of a straight chain alkane as the substrate, because the present invention enables efficient oxidation even of straight chain alkanes, which are hardly oxidized efficiently by conventional methods.

The reaction substrate hydrocarbon may be diluted to an appropriate concentration with a solvent that is inert to the reaction, before use. The present invention, however, can allow the oxidation reaction to proceed even when approximately no solvent is used, because the oxidizer oxygen is used in the coexistence of ozone. The use of approximately no solvent eliminates or minimizes the need for separation of the reaction product oxide from the solvent and contributes to simplification of the production process.

Non-limiting examples of the solvent include organic acids such as acetic acid and propionic acid; nitriles such as acetonitrile, propionitrile, and benzonitrile; amides such as formamide, acetamide, dimethylformamide, and dimethylacetamide; halogenated hydrocarbons such as chloroform, dichloromethane, dichloroethane, carbon tetrachloride, chlorobenzene, and trifluoromethylbenzene; nitro compounds such as nitrobenzene, nitromethane, and nitroethane; esters such as ethyl acetate and butyl acetate; and mixtures of these solvents.

From the viewpoint of reaction efficiency, the liquid to be fed to the gas-liquid mixing unit 5 (or the flow reactor 7) may contain the reaction substrate hydrocarbon in a concentration of preferably 30 weight percent or more, more preferably 50 weight percent or more, furthermore preferably 85 weight percent or more, and particularly preferably 95 weight percent or more.

Oxidation Catalyst

The oxidation catalyst for use in the present invention is not limited, as long as having catalysis on the oxidation reaction, but is preferably selected from catalysts each including a transition metal in the form of an elementary substance, a compound, or an ion, and an inorganic support onto which the transition metal is supported or immobilized. From the viewpoint of reaction rate, the oxidation catalyst is preferably selected from catalysts each including a transition metal compound supported on a support having a Hammett acidity function $(H_0)$ of -9 or less; and catalysts each including a transition metal ion, and a support having a Hammett acidity function $(H_0)$ of -9 or less and being ion-exchanged with the transition metal ion.

The transition metal is often selected from metal elements belonging to Groups 3 to 12 of the periodic table. Non-limiting examples of the metal elements include, of the periodic table, Group 3 elements such as Sc, lanthanoids, and actinoids; Group 4 elements such as Ti, Zr, and Hf; Group 5 elements such as V; Group 6 elements such as Cr, Mo, and W; Group 7 elements such as Mn; Group 8 elements such as Fe and Ru; Group 9 elements such as Co and Rh; Group 10 elements such as Ni, Pd, and Pt; Group 11 elements such as Cu; and Group 12 elements such as Zn. Preferred, but non-limiting examples of the metal elements include elements belonging to Groups 5 to 11 of the periodic table and are typified by elements belonging to Groups 5 to 9, such as Co, Mn, Fe, V, and Mo. The metal element may have any valence not limited and has a valence of typically 0 to about 6, and preferably 2 or 3.

Examples of the transition metal compound include, of the metal elements, inorganic compounds and organic compounds. Non-limiting examples of the inorganic compounds include elementary substances, hydroxides, oxides (including complex oxides), halides (fluorides, chlorides, bromides, and iodides), oxoacid salts (such as nitrates, sulfates, phosphates, borates, and carbonates), isopolyacid salts, and heteropolyacid salts. Non-limiting examples of the organic compounds include complexes, and organic acid salts exemplified by acetates, propionates, cyanides, naphthenates, and stearates; alkylsulfonates such as methanesulfonates, ethanesulfonates, octanesulfonates, and dodecanesulfonates, of which $C_6$-$C_{18}$ alkylsulfonates are typified; and optionally alkyl-substituted arylsulfonates such as benzenesulfonates, p-toluenesulfonates, naphthalenesulfonates, decylbenzenesulfonates, and dodecylbenzenesulfonates, of which $C_6$-$C_{18}$ alkyl-arylsulfonates are typified. Non-limiting examples of the ligands constituting the complexes include OH (hydroxo), alkoxys (such as methoxy, ethoxy, propoxy, and butoxy), acyls (such as acetyl and propionyl), alkoxycarbonyls (such as methoxycarbonyl and ethoxycarbonyl), acetylacetonato, cyclopentadienyl, halogen atoms (such as chlorine and bromine), CO, CN, oxygen atom, $H_2O$ (aquo), phosphorus compounds exemplified by phosphines (such as triphenylphosphine and other triarylphosphines), and nitrogen-containing compounds such as $NH_3$ (ammine), NO, $NO_2$ (nitro), $NO_3$ (nitrato), ethylenediamine, diethylenetriamine, pyridine, and phenanthroline.

Specific, but non-limiting examples of the transition metal compound include, when taking cobalt compounds as an example, divalent or trivalent cobalt compounds exemplified by inorganic compounds such as cobalt hydroxides, cobalt oxides, cobalt chlorides, cobalt bromides, cobalt nitrates, cobalt sulfates, and cobalt phosphate; organic acid salts such as cobalt acetate, cobalt naphthenate, and cobalt stearate; and complexes such as acetylacetonatocobalt. Non-limiting examples of manganese compounds include divalent to pentavalent manganese compounds exemplified by inorganic compounds such as manganese hydroxides, manganese oxides, manganese chlorides, and manganese sulfates; and complexes such as acetylacetonatomanganese. Non-limiting examples of compounds of other transition metal elements include compounds corresponding to the cobalt or manganese compounds. The oxidation catalyst may include each of different transition metal compounds alone or in combination. The combination use of two or more different transition metal compounds having different valences (such as the combination use of a divalent metal compound and a trivalent metal compound) is also preferred. The transition metal compound for use in the present invention preferably includes one or more compounds selected from compounds containing at least one transition metal selected from the group consisting of Co (cobalt), Mn (manganese), Fe (iron), Zr (zirconium), Ce (cerium), V (vanadium), and Mo (molybdenum), more preferably includes one or more compounds selected from cobalt compounds and manganese compounds (of which organic acid salts are preferred), and particularly preferably includes both a cobalt compound and a manganese compound in combination. This is preferred for restraining deterioration in catalytic activity.

The transition metal ion preferably includes one or more ions selected typically from cobalt ions, manganese ions, iron ions, zirconium ions, and cerium ions, and particularly preferably includes a cobalt ion, or a manganese ion, or both in combination (a cobalt ion in combination with a manganese ion).

In an embodiment, the catalyst includes the transition metal in the form of an elementary substance, a compound, or an ion, and an inorganic support onto which the transition metal is supported or immobilized. Non-limiting examples of the "inorganic support" in this catalyst include zeolite, silica ($SiO_2$), alumina ($Al_2O_3$), silica-alumina ($SiO_2$-$Al_2O_3$), and activated carbon.

The support having a Hammett acidity function ($H_0$) of -9 or less has only to have a Hammett acidity function ($H_0$) of -9 or less at least at the surface. Non-limiting examples of such support include sulfuric acid-supporting solids which are solids (such as $Al_2O_3$, $SiO_2$, zeolite, $SiO_2$-$Al_2O_3$, polymers, graphite, and metals) supporting concentrated sulfuric acid; solid strong acids; and solid super strong acids (acids having a Hammett acidity function ($H_0$) of less than -11.93). The concentrated sulfuric acid has a Hammett acidity function $H_0$ of -9.88 at 96%, of -10.27 at 98%, and of -11.93 at 100%.

Non-limiting examples of the solid strong acids and solid super strong acids include immobilized liquid super strong acids each containing a liquid super strong acid (such as $SbF_5$, $BF_3$, $BF$-$SbF_5$, $FSO_3H$-$SbF_5$, or $TaF_5$) supported on a solid (such as $Al_2O_3$, $SiO_2$, zeolite, $SiO_2$-$Al_2O_3$, a polymer, graphite, or a metal); binary metal salts each prepared by grinding and mixing $AlCl_3$ or $AlBr_3$ with one selected from $CuSO_4$, $CuCl_2$, $Ti_2(SO_4)_3$, and $TiCl_3$; sulfated metal oxides each including a sulfate ion supported by and bonded to a metal oxide (such as $Fe_2O_3$, $TiO_2$, $ZrO_2$, $HfO_2$, $SnO_2$, $Al_2O_3$, or $SiO_2$) via adsorption and firing; noble metals/sulfated metal oxides each including the sulfated metal oxide mixed with a noble metal such as Ir or Pt; metal oxide super strong acids formed by allowing a metal oxide (such as $ZrO_2$, $SnO_2$, $TiO_2$, or $Fe_2O_3$) to adsorb, for example, $WO_3$, $MoO_3$, or $B_2O_3$, and firing the resulting article at a high temperature; strongly acidic or super acidic ion exchange resins, exemplified by non-porous or porous ion exchange resins containing a strong acid group or superacid group such as -$CF_3$, $CF_2$, or $SO_3H$; and heteropolyacids such as polyacids containing an element selected typically from P, Mo, V, W, and Si. Non-limiting examples of the strongly acidic or super acidic ion exchange resins include fluorinated sulfonic acid resins (fluorocarbon resins containing a sulfo-containing group in a side chain). Preferred, but non-limiting examples of the fluorinated sulfonic acid resins include copolymers between a sulfo-containing perfluorovinyl ether monomer and tetrafluoroethylene, such as Nafion® NR50 (supplied by Aldrich) and Nafion® H (supplied by E. I. du Pont de Nemours & Co.).

In particular, the support for use in the present invention is preferably selected from sulfated metal oxides, noble metals/sulfated metal oxides, metal oxide super strong acids, and strongly acidic or super acidic ion exchange resins (such as fluorinated sulfonic acid resins), and particularly preferably selected from sulfated metal oxides, noble metals/sulfated metal oxides, and fluorinated sulfonic acid resins. The sulfated metal oxides, noble metals/sulfated metal oxides, and fluorinated sulfonic acid resins have particularly high acid strengths and can thereby more effectively support the transition metal compound. Of the sulfated metal oxides, preferred examples include sulfated zirconia, sulfated tin, and sulfated hafnium. Of the noble metals/sulfated metal oxides, preferred examples include Pt/sulfated zirconia, Ir/sulfated zirconia, and Pd/sulfated zirconia. Among them, the support is advantageously selected from sulfated zirconia and fluorinated sulfonic acid resins, each of which is industrially readily available. In particular, the support is preferably selected from fluorinated sulfonic acid resins (such as Nafion® and other copolymers between a sulfo-containing perfluorovinyl ether monomer and tetrafluoroethylene).

The support for use in the present invention is not limited in shape and particle size and can be selected from those having an appropriate shape and particle size suitable for the flow reactor. Non-limiting examples of the shape include shapes of pellets, powders, films, coatings (layers), and any other shapes generally employed as solid catalysts.

The supporting of the transition metal in the form of an elementary substance, a compound, or an ion onto the support (such as an inorganic support, a sulfuric acid-supporting solid, or a solid super strong acid) may be performed by any of common processes such as impregnation, firing, precipitation, and ion exchange processes.

The transition metal compound may be supported in an amount of typically about 0.001 to about 20 weight percent, preferably about 0.01 to about 20 weight percent, and particularly about 0.1 to about 10 weight percent, in terms of the metal atom in the transition metal compound, relative to the weight of the support (such as an inorganic support, a sulfuric acid-supporting solid, or a solid super strong acid). When the support is ion-exchanged with the transition metal ion, the ion-exchange amount is typically about 0.001 to about 20 weight percent, preferably about 0.01 to about 20 weight percent, and particularly about 0.1 to about 10 weight percent, in terms of the metal atom in the transition metal ion, relative to the weight of the support (such as a sulfuric acid-supporting solid or a solid super strong acid).

The oxidation catalyst in the present invention is immobilized to or packed in the flow reactor. The immobilization or packing of the oxidation catalyst may be performed by a process publicly known or well known in the art. The oxidation catalyst may be packed into space in the flow reactor, or may be immobilized typically as a coating to the inner wall of the flow reactor.

In particular, in a preferred embodiment in the present invention, a coating is disposed on the inner wall of the flow reactor, where the coating includes a sulfuric acid-supporting solid or solid super strong acid being ion-exchanged with the transition metal ion. Especially, in a particularly preferred embodiment, a coating of a strongly acidic or super acidic ion exchange resin being ion-exchanged with the transition metal ion is disposed on the inner wall of the flow reactor. The flow reactor as mentioned above may be produced typically by coating the inner wall surface of a tube typically with a strongly acidic or super acidic ion exchange resin (such as a fluorinated sulfonic acid resin) and subjecting the resulting article to ion exchange with a transition metal compound solution (such as a cobalt compound solution). The tube may be made typically of a resin, glass, or a metal. The tube is preferably selected from tubes made of fluorocarbon resins such as polytetrafluoroethylenes (PTFEs).

In an embodiment, the monolithic catalyst reactor is used as the flow reactor. In a preferred embodiment in this case, a transition metal in the form of an elementary substance, a compound, or an ion is supported on the skeleton (material portion) of the monolith support. Examples of the transition metal are as mentioned above, of which at least one selected from Co, Mn, Fe, Zr, Ce, V, and Mo is preferred, and at least one selected from Co and Mn is particularly preferred. The monolithic catalyst reactor according to this embodiment can be prepared typically by allowing a solution of a transition metal compound to flow through the monolith support and thereby allowing the transition metal to be supported by the monolith support. In the embodiment where the monolithic catalyst reactor is used as the flow reactor, an embodiment as above is also preferred, in which a coating including a strongly acidic or super acidic ion exchange resin (such as a fluorinated sulfonic acid resin) being ion-exchanged with a transition metal ion is disposed on the skeleton (material portion) of the monolith support.

Imide Compound Having Cyclic Imide Skeleton

An imide compound having a cyclic imide skeleton may be used as a promoter in the present invention, so as to further promote the oxidation reaction. Specifically, the oxidation reactor may further include an imide compound inlet channel upstream from the flow reactor, where the imide compound having a cyclic imide skeleton is introduced through the imide compound inlet channel.

The imide compound having a cyclic imide skeleton for use herein can be selected from a variety of imide compounds having a cyclic imide skeleton, which are known as oxidation catalysts.

Of such imide compounds having a cyclic imide skeleton, representative, but non-limiting examples of compounds having a 5-membered cyclic imide skeleton include 5-membered cyclic N-hydroxyimide compounds such as N-hydroxysuccinimide, N-hydroxy-$\alpha$-methylsuccinimide, N-hydroxy-$\alpha$,$\alpha$-dimethylsuccinimide, N-hydroxy-$\alpha$,$\beta$-dimethylsuccinimide, N-hydroxy-$\alpha$,$\alpha$,$\beta$,$\beta$-tetramethylsuccinimide, N-hydroxymaleimide, N-hydroxyhexahydrophthalimide, N,N'-dihydroxycyclohexanetetracarboxylic diimide, N-hydroxyphthalimide, N-hydroxytetrabromophthalimide, N-hydroxytetrachlorophthalimide, HET acid N-hydroxyimide (N-hydroxy-1,4,5,6,7,7-hexachlorobicyclo[2.2.1]hept-5-ene-2,3-dicarboximide), himic acid N-hydroxyimide (N-hydroxy-bicyclo[2.2.1]hept-5-ene-2,3-dicarboximide, N-hydroxytrimellitimide, N,N'-dihydroxypyromellitic diimide, N,N'-dihydroxynaphthalenetetracarboxylic diimide, $\alpha$,$\beta$-diacetoxy-N-hydroxysuccinimide, N-hydroxy-$\alpha$,$\beta$-bis(propionyloxy)succinimide, N-hydroxy-$\alpha$,$\beta$-bis(valeryloxy)succinimide, N-hydroxy-$\alpha$,$\beta$-bis(lauroyloxy)succinimide, $\alpha$,$\beta$-bis(benzoyloxy)-N-hydroxysuccinimide, N-hydroxy-4-methoxycarbonylphthalimide, 4-ethoxycarbonyl-N-hydroxyphthalimide, N-hydroxy-4-pentyloxycarbonylphthalimide, 4-dodecyloxy-N-hydroxycarbonylphthalimide, N-hydroxy-4-phenoxycarbonylphthalimide, N-hydroxy-4,5-bis(methoxycarbonyl)phthalimide, 4,5-bis(ethoxycarbonyl)-N-hydroxyphthalimide, N-hydroxy-4,5-bis(pentyloxycarbonyl)phthalimide, 4,5-bis(dodecyloxycarbonyl)-N-hydroxyphthalimide, and N-hydroxy-4,5-bis(phenoxycarbonyl)phthalimide; and compounds corresponding to these compounds, except with a hydroxy group or groups bonded to the nitrogen atom being protected with a protecting group or groups.

Non-limiting examples of the protecting groups for the hydroxy groups include acyls such as acetyl, propionyl, and benzoyl; groups capable of forming an acetal or hemiacetal bond with the hydroxy group or groups; sulfonyls such as methanesulfonyl and p-toluenesulfonyl; and groups each resulting from removing a OH group from an inorganic acid.

Of the imide compounds having a cyclic imide skeleton, representative, but non-limiting examples of compounds having a 6-membered cyclic imide skeleton include 6-membered cyclic N-hydroxyimide compounds such as N-hydroxyglutarimide, N-hydroxy-$\alpha$,$\alpha$,-dimethylglutarimide, N-hydroxy-$\beta$,$\beta$-dimethylglutarimide, N-hydroxy-1,8-decahydronaphthalenedicarboximide, N,N'-dihydroxy-1,8;4,5-decahydronaphthalenetetracarboxylic diimide, N-hydroxy-1,8-naphthalenedicarboximide (N-hydroxynaphthalimide), and N,N'-dihydroxy-1,8;4,5-naphthalenetetracarboxylic diimide; and compounds corresponding to these compounds, except with a hydroxy group or groups bonded to the nitrogen atom being protected by a protecting group or groups. The protecting groups for the hydroxy groups are as described above.

Particularly preferred, but non-limiting examples of the imide compound having a cyclic imide skeleton include N-hydroxysuccinimide, N-hydroxyphthalimide, N,N'-dihydroxypyromellitic diimide, N-hydroxyglutarimide, N-hydroxy-1,8-naphthalenedicarboximide, and N,N'-dihydroxy-1,8;4,5-naphthalenetetracarboxylic diimide; and compounds resulting from introducing the protecting group or groups into the hydroxy group or groups of these N-hydroxyimide compounds.

Each of different imide compounds having a cyclic imide skeleton may be used alone or in combination.

The imide compound having a cyclic imide skeleton may be used in an amount of typically about 0.0000001 to about 1 mole, preferably 0.00001 to 0.5 mole, and particularly preferably 0.0001 to 0.4 mole, per mole of the reaction substrate hydrocarbon. The imide compound having a cyclic imide skeleton, when used in an amount within the range, can allow the oxidation reaction to proceed at a high reaction rate.

The imide compound having a cyclic imide skeleton may be fed in the form of a solution in a solvent to the reaction system. The solvent for use herein may be selected from solvents inert to the reaction, such as the solvents described in relation to the reaction substrate.

The oxidation reactor according to the present invention may further include a transition metal compound inlet channel upstream from the flow reactor. Through the transition metal compound inlet channel, a solution containing the transition metal compound is introduced so as to make up for the transition metal compound or the transition metal ion in the oxidation catalyst immobilized to or packed in the flow reactor. The solvent in the solution containing the transition metal compound may be selected from solvents inert to the reaction, such as the solvents described in relation to the reaction substrate.

Oxidation Reaction

The reaction temperature in the oxidation reaction herein can be selected as appropriate according typically to the types of the reaction substrate hydrocarbon and of the target product, but is typically around room temperature to around 200°C, preferably 50°C to 130°C, and particularly preferably 60°C to 100°C. The reaction can be performed at normal atmospheric pressure or under pressure (under a load). When the reaction is performed under pressure (under a load), the reaction pressure is generally about 0.1 to about 10 MPa, preferably 0.15 to 8 MPa, and particularly preferably 0.5 to 8 MPa. The present invention allows the oxidation reaction to proceed smoothly even at normal atmospheric pressure (0.1 MPa), because of using the oxidizer oxygen in combination with ozone.

To the gas-liquid mixing unit 5, the gas is fed through the gas inlet channel 4, and the liquid is fed through the liquid inlet channel 15. The ratio of the flow rate (mL/min) of the gas to the flow rate (mL/min) of the liquid herein has only to be such a ratio that a gas-liquid mixture is formed and the reaction proceeds efficiently. However, the ratio is generally from 2:8 to 8:2, preferably from 3:7 to 7:3, and more preferably from 4:6 to 6:4.

From the points typically of reaction efficiency and thermal efficiency, the gas-liquid mixture in the flow reactor may flow at a linear velocity (LV) of typically 50 to 10000 cm/min, preferably 100 to 5000 cm/min, and more preferably 100 to 1000 cm/min.

The linear velocity (LV) (cm/min) of the gas-liquid mixture may be determined according to the expression:

$$LV = (Q1 + Q2)/S$$

where:

Q1 represents the gas supply amount (the flow rate of the gas flowing through the gas inlet channel 4) ($cm^3$/min);
Q2 represents the liquid supply amount (the flow rate of the liquid flowing through the liquid inlet channel 15) ($cm^3$/min); and
S represents the cross-sectional area ($cm^2$) of the flow reactor.

The area S is determined according to the expression:

$$S = (D/2)^2 \times \pi$$

where
D represents the inside diameter (cm) of the flow reactor, where, when a coating is disposed on the inner wall, the term "inside diameter" refers to an inside diameter excluding the coating thickness.

A reaction mixture discharged from the flow reactor 7 is separated into a gas and a liquid in the gas-liquid separator 9, as described above. The separated liquid is recovered through the liquid recovery channel 16. When the one-pass conversion is low, at least part of the liquid may be recycled to the reaction system. In an embodiment, the reaction liquid may be recovered through the liquid recovery channel 16 after performing the operation of recycling the liquid separated in the gas-liquid separator 9 to the reaction system for a predetermined time. In another embodiment, a continuous system may be employed, in which part of the liquid separated in the gas-liquid separator 9 is continuously or intermittently drawn out (recovered) through the liquid recovery channel 16, while the remainder of the liquid is recycled to the reaction system.

The target oxide or oxides can be yielded by subjecting the recovered liquid to a separation process such as filtration, concentration, distillation, extraction, crystallization, recrystallization, or column chromatography, or a separation process as any combination of them.

Examples

The present invention will be illustrated in further detail with reference to several examples below. It should be noted, however, that the examples are by no means intended to limit the scope of the present invention.

Example 1

Tetradecane was oxidized according to the flow illustrated in Fig. 1. Initially, the oxidation reactor will be described. Oxygen is fed from an oxygen cylinder (not shown) through the oxygen inlet channel 2 to the ozone generator 3 and partially converted into ozone. Into this, nitrogen is introduced from a nitrogen supply line (not shown) to form an oxygen-ozone-nitrogen mixed gas (mixed gas containing oxygen, ozone, and nitrogen). The mixed gas is fed through the gas inlet channel 4 to the microbubble generator 50 (see Fig. 2). The microbubble generator 50 acts as a gas-liquid mixer (gas-liquid mixing unit) and is made of steel-use-stainless (SUS). Independently, the reaction substrate tetradecane (liquid) is fed through the hydrocarbon supply channel 1 to the microbubble generator 50. However, the hydrocarbon supply channel 1 was not used in this example. Fig. 2 is a schematic view of the microbubble generator 50 when viewed laterally. The microbubble generator used in this example was the "V Type Microbubble Nozzle" supplied by Kansai Automation Equipment Co., Ltd. The microbubble generator 50 illustrated in Fig. 2 has or includes the upstream side (liquid inlet side) 51, the inlet port (gas inlet side) 52, the downstream side (microbubble-containing liquid outlet side) 53, the nozzle 54, and the diffuser 55. The arrows in the figure indicate introduction directions of the liquid and the gas. The microbubble generator includes a cylindrical channel extending from the upstream side 51 to the downstream side 53, which channel is narrowed in inside diameter at a midpoint thereof. To the narrowed portion (the tip of the nozzle 54), a gas channel from the inlet port 52 is perpendicularly coupled. The liquid introduced from the upstream side 51 flows, with the gas (mixed gas containing oxygen and ozone) introduced from the inlet port 52, from the nozzle 54 through the most-narrowed portion and gushes forth toward the diffuser 55 to form a microbubble-containing liquid. The formed microbubble-containing liquid is discharged from the downstream side 53. The cylindrical channel extending from the upstream side 51 to the downstream side 53 has an inside diameter of 1/2 inch, an inside diameter in the narrowed portion of 1/6 inch, and a length of 3 inch. The gas channel from the inlet port 52 has an inside diameter of 3/16 inch. The inlet port 52 is coupled to the gas inlet channel 4; the upstream side 51 is coupled to the liquid supply channel 15; and the downstream side 53 is coupled to the gas-liquid mixture channel 6 mentioned below.

The oxygen-ozone-nitrogen mixed gas introduced through the inlet port 52 into the microbubble generator 50 is converted into microbubbles in the cylindrical channel, mixed with tetradecane introduced from the upstream side 51 via gas-liquid mixing, discharged from the downstream side 53, and forwarded through the gas-liquid mixture channel 6 to the flow reactor 7.

The flow reactor 7 was prepared in the following manner. Specifically, a 20 weight percent Nafion dispersion (supplied by E. I. du Pont de Nemours & Co.) was allowed to flow through a Teflon® tube having an inside diameter of 2.0 mm and a length of 1.5 m, the resulting article was dried at about 100°C, and yielded a Teflon® tube bearing a 0.5-mm thick Nafion coating on the inner wall. The resulting tube had an inside diameter of 1.0 mm. Through this tube, a 5 weight percent solution of cobalt acetate ($Co(OAc)_2$) in acetic acid was allowed to flow, the resulting article was heated at about 100°C for one hour, dried at 120°C, and yielded a flow reactor bearing, on the inner wall, a coating of a fluorinated sulfonic acid resin being ion-exchanged with cobalt ions. The fluorinated sulfonic acid resin coating has a cobalt concentration of 2.4 weight percent. All (100%) of ion exchange groups of Nafion are replaced (exchanged) with cobalt ions.

Part of the flow reactor 7 corresponding to a length of 1 m is immersed in an oil bath (at 90°C) equipped with a temperature controller. A reaction mixture discharged from the flow reactor 7 is introduced through the reaction mixture channel 8 into the gas-liquid separator 9 and is separated into a gas and a liquid, where the gas-liquid separator 9 is a steel-use stainless (SUS) vessel having an inner capacity of 100 mL. The separated gas is introduced into the ozone decomposer 12 and then discharged through the exhaust gas line 13 to the outside. Where necessary, part of the separated gas is recycled through the gas circulation channel 11 to the reaction system. However, the gas was not recycled in this example. Independently, the liquid separated in the gas-liquid separator 9 can be recycled through the liquid circulation channel 10 and the liquid inlet channel 15 to the microbubble generator 50, using a pump for high-performance liquid chromatographs (not shown). The liquid separated in the gas-liquid separator 9 can also be recovered through the liquid recovery channel 16. As needed, an imide compound having a cyclic imide skeleton may be fed through the imide compound inlet channel 14 and the liquid inlet channel 15 to the microbubble generator 50. However, the imide compound was not used in this example.

Using the oxidation reactor, the oxidation reaction of tetradecane was performed. Specifically, 20 g of tetradecane were placed in the gas-liquid separator 9 and heated up to 90°C with stirring in a nitrogen atmosphere. Oxygen was fed through the oxygen inlet channel 2 to the ozone generator 3, partially converted into ozone, and mixed with nitrogen to give an oxygen-ozone-nitrogen mixed gas containing 20.5 volume percent of oxygen, 0.5 volume percent of ozone, and 79.0 volume percent of nitrogen. The mixed gas was fed at a flow rate of 10 mL/min through the gas inlet channel 4 to the microbubble generator 50. Independently, tetradecane in the gas-liquid separator 9 was fed at a flow rate of 10 mL/min through the liquid circulation channel 10 and the liquid inlet channel 15 to the microbubble generator 50. The oxygen-ozone-nitrogen mixed gas fed to the microbubble generator 50 was converted into microbubbles in the cylindrical channel extending from the upstream side to the downstream side, mixed with tetradecane via gas-liquid mixing, discharged from the downstream side 53, and forwarded through the gas-liquid mixture channel 6 to the flow reactor 7. A reaction mixture discharged from the flow reactor 7 was fed through the reaction mixture channel 8 to the gas-liquid separator 9 and separated into a gas and a liquid. The gas separated in the gas-liquid separator 9 was introduced into the ozone decomposer 12 and discharged through the exhaust gas line 13 to the outside. The liquid separated in the gas-liquid separator 9 was recycled through the liquid circulation channel 10 and the liquid inlet channel 15 to the microbubble generator 50, using the pump. After performing this operation for 8 hours, the pump was stopped, and the composition (formulation) of a liquid in the gas-liquid separator 9 was analyzed by gas chromatography (column: DB-1, supplied by Agilent Technologies) to find that the conversion from tetradecane was 17.5%; and that tetradecanol, tetradecanone, and tetradecanoic acid were formed as oxides (oxidation products) of tetradecane.

Example 2

An operation as in Example 1 was performed, except for using a monolithic column (supplied by Kyoto Monotech Co., Ltd., trade name MonoBis, high-pressure type) as the flow reactor 7. The monolithic column had a diameter of 3.2 mm and a length of 150 mm and was made of porous silica.

On the monolithic column, a metal catalyst was supported in the following manner, to give a monolithic catalyst. Specifically, a 5 weight percent cobalt acetate $(Co(OAc)_2)$ in acetic acid was allowed to flow through the monolithic column, the resulting article was heated at about 100°C for one hour, dried at 120°C, and yielded a monolithic catalyst reactor including cobalt supported on the porous silica surface of the monolithic column. Cobalt was supported in an amount of 1.2 weight percent relative to the monolith support (porous silica).

This monolithic catalyst reactor is immersed in an oil bath (at 90°C) equipped with a temperature controller. A reaction mixture discharged from the monolithic catalyst reactor is introduced through the reaction mixture channel 8 into the gas-liquid separator 9 and separated into a gas and a liquid. The gas-liquid separator 9 is a steel-use-stainless (SUS) vessel having an inner capacity of 100 mL. The separated gas is introduced into the ozone decomposer 12 and then discharged through the exhaust gas line 13 to the outside. Part of the separated gas is, as needed, recycled through the gas circulation channel 11 to the reaction system. However, the gas was not recycled in this example. Independently, the liquid separated in the gas-liquid separator 9 can be recycled through the liquid circulation channel 10 and the liquid inlet channel 15 to the microbubble generator 50, using a pump for high-performance liquid chromatographs (not shown). The liquid separated in the gas-liquid separator 9 can also be recovered through the liquid recovery channel 16. As needed, an imide compound having a cyclic imide skeleton may be fed through the imide compound inlet channel 14 and the liquid inlet channel 15 to the microbubble generator 50. However, the imide compound was not used in this example.

Using the oxidation reactor, the oxidation reaction of tetradecane was performed. Specifically, 20 g of tetradecane were placed in the gas-liquid separator 9 and heated up to 90°C with stirring in a nitrogen atmosphere. Oxygen was fed through the oxygen inlet channel 2 to the ozone generator 3, partially converted into ozone, mixed with nitrogen, and yielded an oxygen-ozone-nitrogen mixed gas containing 20.5 volume percent of oxygen, 0.5 volume percent of ozone, and 79.0 volume percent of nitrogen. The mixed gas was fed at a flow rate of 10 mL/min through the gas inlet channel 4 to the microbubble generator 50. Independently, tetradecane in the gas-liquid separator 9 was fed at a flow rate of 10 mL/min through the liquid circulation channel 10 and the liquid inlet channel 15 to the microbubble generator 50. The oxygen-ozone-nitrogen mixed gas fed to the microbubble generator 50 was converted into microbubbles in the cylindrical channel extending from the upstream side to the downstream side, mixed with tetradecane via gas-liquid mixing, discharged from the downstream side 53, and forwarded through the gas-liquid mixture channel 6 to the monolithic catalyst reactor. A reaction mixture discharged from the monolithic catalyst reactor was fed through the reaction mixture channel 8 to the gas-liquid separator 9 and separated into a gas and a liquid. The gas separated in the gas-liquid separator 9 was introduced into the ozone decomposer 12 and discharged through the exhaust gas line 13 to the outside. The liquid separated in the gas-liquid separator 9 was recycled through the liquid circulation channel 10 and the liquid inlet channel 15 to the microbubble generator 50, using the pump. After performing this operation for 8 hours, the pump was stopped, and the composition (formulation) of a liquid in the gas-liquid separator 9 was analyzed by gas chromatography (column: DB-1, supplied by Agilent Technologies) to find that the conversion from tetradecane was 30.5%; and that tetradecanol, tetradecanone, and tetradecanoic acid were formed as oxides of tetradecane.

Comparative Example 1

Tetradecane oxidation was performed by a procedure as in Example 1, except for using a commercially available T mixer (made of Teflon®, supplied by Flonchemical Co., Ltd., having an inside diameter of 1.0 mm) instead of the gas-liquid mixer 5 (the microbubble generator 50). The T-mixer was configured so that the mixed gas was fed from the lower part of "T", tetradecane was fed from the left hand of "T", the two components were merged at the crossing of "T", and the merged fluid was allowed to flow toward the right hand of "T".
As a result, it was found that the conversion from tetradecane was 2.0%; and that tetradecanol, tetradecanone, and tetradecanoic acid were formed as oxidation products.

Example 2

An operation as in Example 1 was performed, except for using 2,2,4-trimethylpentane as a reaction substrate instead of tetradecane. As a result, the substrate conversion was found to be 25%.

Example 3

An operation as in Example 1 was performed, except for using n-heptane as a reaction substrate instead of tetradecane. As a result, the substrate conversion was found to be 24%.

Example 4

An operation as in Example 1 was performed, except for using toluene as a reaction substrate instead of tetradecane. As a result, the substrate conversion was found to be 100%.

Example 5

An operation as in Example 1 was performed, except for using 2-methylbiphenyl as a reaction substrate instead of tetradecane. As a result, the substrate conversion was found to be 98.0%.

Industrial Applicability

According to the present invention, a monolithic catalyst reactor is used as the flow reactor, where the monolithic catalyst reactor includes a monolith support, and an oxidation catalyst immobilized to or packed in the monolith support. In addition or alternatively, the gasliquid mixing unit includes a microbubble generator. Further, both oxygen and ozone are used as oxidizers. This configuration allows reaction components to move quickly and allows oxidation to proceed rapidly at a high rate. Accordingly, with the present invention, a wide variety of hydrocarbons, in particular even straight chain alkanes, can be efficiently oxidized. The reaction heat can be efficiently removed, and high energy efficiency is obtained, because of smooth heat exchange. In addition and advantageously, the oxidation apparatus can be downsized.

Reference Signs List

1 hydrocarbon supply channel
2 oxygen inlet channel
3 ozone generator
4 gas inlet channel
5 gas-liquid mixing unit (gas-liquid mixer)
6 gas-liquid mixture channel
7 flow reactor
8 reaction mixture channel
9 gas-liquid separator
10 liquid circulation channel
11 gas circulation channel
12 ozone decomposer
13 exhaust gas line
14 imide compound inlet channel
15 liquid inlet channel
16 liquid recovery channel
50 microbubble generator
51 upstream side (liquid inlet side)
52 inlet port (gas inlet side)
53 downstream side (microbubble-containing liquid outlet side)
54 nozzle
55 diffuser

**Claims**

1. An oxidation reactor comprising:

    a liquid inlet channel through which a liquid containing a reaction substrate hydrocarbon is introduced;
    a gas inlet channel through which a gas containing oxygen and ozone is introduced;
    a gas-liquid mixing unit that mixes the liquid introduced from the liquid inlet channel with the gas introduced from the gas inlet channel; and
    a flow reactor in which an oxidation catalyst is immobilized or packed,
    the flow reactor comprising:

        a monolith support; and
        the oxidation catalyst immobilized to or packed in the monolith support, and/or

the gas-liquid mixing unit comprising a microbubble generator.

2. The oxidation reactor according to claim 1, further comprising
a gas-liquid separator downstream from the flow reactor.

3. The oxidation reactor according to claim 2, further comprising
a circulation channel through which at least part of a liquid separated in the gas-liquid separator is recycled to the gas-liquid mixing unit or to a portion upstream from the gas-liquid mixing unit.

4. The oxidation reactor according to one of claims 2 and 3, further comprising
a circulation channel through which at least part of a gas separated in the gas-liquid separator is recycled to the gas-liquid mixing unit or to a portion upstream from the gas-liquid mixing unit.

5. The oxidation reactor according to any one of claims 1 to 4,
wherein the oxidation catalyst is a catalyst comprising:

a transition metal in a form of an elementary substance, a compound, or an ion; and
an inorganic support onto which the transition metal is supported or immobilized.

6. The oxidation reactor according to any one of claims 1 to 4,
wherein the oxidation catalyst is selected from:

a catalyst comprising:

a transition metal compound; and
a support having a Hammett acidity function $(H_0)$ of -9 or less and supporting the transition metal compound, and a catalyst comprising:

a transition metal ion; and
a support having a Hammett acidity function $(H_0)$ of -9 or less and being ion-exchanged with the transition metal ion.

7. The oxidation reactor according to claim 6,
wherein the support comprises

a strongly acidic or super acidic ion exchange resin.

8. The oxidation reactor according to any one of claims 1 to 4, 6, and 7,
wherein the flow reactor comprises

a coating on its inner wall, the coating comprising:

a transition metal ion; and
a strongly acidic or super acidic ion exchange resin being ion-exchanged with the transition metal ion.

9. The oxidation reactor according to any one of claims 1 to 8, further comprising
an imide compound inlet channel upstream from the flow reactor, where an imide compound having a cyclic imide skeleton is introduced through the imide compound inlet channel.

10. A method for producing an oxide, the method comprising
oxidizing a hydrocarbon in the coexistence of oxygen and ozone using the oxidation reactor according to any one of claims 1 to 9 to yield a corresponding oxide.

[Fig. 1]

[Fig. 2]

## INTERNATIONAL SEARCH REPORT

| International application No. |
|---|
| PCT/JP2015/072504 |

| A. CLASSIFICATION OF SUBJECT MATTER |
|---|
| C07C27/16(2006.01)i, C07C29/48(2006.01)i, C07C31/125(2006.01)i, C07C45/40 (2006.01)i, C07C49/04(2006.01)i, C07C51/34(2006.01)i, C07C53/126 (2006.01)i, B01F3/04(2006.01)n, B01J32/00(2006.01)n, C07B61/00(2006.01)n |
| According to International Patent Classification (IPC) or to both national classification and IPC |

| B. FIELDS SEARCHED |
|---|
| Minimum documentation searched (classification system followed by classification symbols) |
| C07C27/00-71/00, B01F3/00-3/22, B01J32/00, C07B61/00 |

| Documentation searched other than minimum documentation to the extent that such documents are included in the fields searched |
|---|
| Jitsuyo Shinan Koho          1922–1996   Jitsuyo Shinan Toroku Koho   1996–2015 |
| Kokai Jitsuyo Shinan Koho    1971–2015   Toroku Jitsuyo Shinan Koho   1994–2015 |

| Electronic data base consulted during the international search (name of data base and, where practicable, search terms used) |
|---|
|  |

## C. DOCUMENTS CONSIDERED TO BE RELEVANT

| Category* | Citation of document, with indication, where appropriate, of the relevant passages | Relevant to claim No. |
|---|---|---|
| A | JP 2006-257001 A  (Mitsubishi Chemical Corp.), 28 September 2006 (28.09.2006), claims; paragraphs [0031], [0037], [0047] to [0049]; examples; drawings (Family: none) | 1-10 |
| A | JP 2008-280328 A  (Sumitomo Chemical Co., Ltd.), 20 November 2008 (20.11.2008), claims; paragraphs [0009], [0013], [0016]; examples & CN 101284760 A        & EP 1980549 A1 & KR 10-2008-0092855 A   & TW 200902487 A & US 2008/0255392 A1 claims; paragraphs [0010], [0014], [0017]; examples | 1-10 |

| ☒  Further documents are listed in the continuation of Box C. | ☐  See patent family annex. |
|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 15 October 2015 (15.10.15) | 27 October 2015 (27.10.15) |

| Name and mailing address of the ISA/ | Authorized officer |
|---|---|
| Japan Patent Office 3-4-3,Kasumigaseki,Chiyoda-ku, Tokyo 100-8915,Japan |  |
|  | Telephone No. |

Form PCT/ISA/210 (second sheet) (July 2009)

**INTERNATIONAL SEARCH REPORT**

International application No.

PCT/JP2015/072504

C (Continuation). DOCUMENTS CONSIDERED TO BE RELEVANT

| Category* | Citation of document, with indication, where appropriate, of the relevant passages | Relevant to claim No. |
|---|---|---|
| A | JP 2008-201723 A (Fuji Xerox Co., Ltd.), 04 September 2008 (04.09.2008), claims; paragraphs [0030] to [0032]; examples; drawings (Family: none) | 1-10 |
| A | WO 2009/048141 A1 (Nippon Soda Co., Ltd. et al.), 16 April 2009 (16.04.2009), claims; paragraph [0018]; examples; drawings & CN 101820995 A & EP 2206550 A1 & JP 5598952 B2 & KR 10-2010-0053679 A & US 2010/0210876 A1 claims; paragraph [0032]; examples; drawings | 1-10 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014175315 A

- JP 2002331242 A